# EUROPEAN PATENT SPECIFICATION

(11) **EP 2 446 840 B1**
(45) Date of publication and mention of the grant of the patent: **27.06.2018**
(21) Application number: 11160023.5
(22) Date of filing: 28.03.2011
(51) Int. Cl.: A61B 17/24, A46B 15/00

(54) **Tongue cleaner**
Zungenreiniger
Produit nettoyant de la langue

(30) Priority: 22.02.2011 JP 2011035359; 08.10.2010 WO PCT/JP2010/067769
(43) Date of publication of application: 02.05.2012
(73) Proprietor: Shikien Co. ltd., Niigata-shi, Niigata 956-0057 (JP)
(72) Inventor: Tanaka, Michio, Niigata-shi Niigata 9560057 (JP)
(74) Representative: Patentanwälte Bauer Vorberg Kayser

(56) References cited:
- WO-A2-02/071967
- DE-A1- 2 455 240
- DE-A1- 10 028 530
- DE-A1-102007 048 877
- GB-A- 1 331 747
- US-A- 2 800 149
- US-A- 3 009 235
- US-A- 3 943 592
- US-A- 5 875 526
- US-A- 5 938 673
- US-A- 6 131 228
- US-A1- 2001 041 903
- US-A1- 2007 178 273
- US-A1- 2007 255 177
- US-A1- 2009 131 960

## Description

The present invention relates to a tongue cleaner. Heretofore, as this sort of tongue cleaner, there has been proposed a tongue cleaner including a head and a stick-shaped handle connected to one end of the head. In the tongue cleaner, the head includes, on one of its front and reverse faces, a concavely curved surface with a valley portion extending parallel to the longitudinal direction of the stick-shaped handle. Further, the head is formed in a flat and thin shape, having a substantially uniform thickness, and the other face of the head is formed into a convexly curved surface. Then, a sheet composed of a twill-woven textile, a sateen-woven textile and a pile-woven textile is attached to both the front and reverse faces of the head. When cleaning tongue coating on both lingual sides using this tongue cleaner, the lingual raised sides are allowed to fit in the concavely curved surface, thus enabling tongue coating to be cleaned without shifting the tongue cleaner laterally, simply by moving the tongue cleaner back and forth with the stick-shaped handle held in hand and the concavely curved surface brought into contact with the tongue.

Further, as an improvement to the above-mentioned conventional art, there has been proposed the one in which said head is configured such that a sheet is provided on a surface of a core material formed with a concavely curved surface, the sheet being fixed on a lateral side of the core material; the concavely curved surface and the sheet opposed to the concavely curved surface are formed so as to be slightly slidable relative to each other; and when moving the tongue cleaner back and forth with its head in contact with a tongue, the grime behind tongue coating can be scraped out while raising tongue coating by the sheet that is slightly slidable relative to the head. At this time, the sheet opposed to each of the concavely curved surface and convexly curved surface becomes slidable, thus enabling the sheet to softly contact with the tongue, while permitting the tongue to be less affected by a possible strong force applied to the stick-shaped handle owing to this sliding motion of the sheet.

Further, heretofore, there has been disclosed another tongue cleaner in which a loop pile fabric whose pile surface is composed of multifilament yarns is fixed to a tip of a stick. Besides, various patent publications disclose tongue cleaners which employ looped piles.
Patent document 1: Japanese unexamined patent application publication JP 2008-188275 A
Patent document 2: US unexamined patent application publication US 2009/131960 A1
Patent document 3: Japanese unexamined utility model publication JP 2515465
Patent document 4: US patent publication US 5, 938, 673 A The preamble of claim 1 is based on US 5,938,673 A.
In the conventional art according to the above-mentioned patent document 2, the convexly curved surface and the sheet opposed to the convexly curved surface are always in close contact with each other so that the sheet is able to slide on the convexly curved surface. In contrast, the concavely curved surface and the sheet opposed to the concavely curved surface are formed with a gap therebetween while it is being in pristine condition, and the sheet is then slightly stretched as a user continues to use the tongue cleaner so that the sheet comes in close contact with the concavely curved surface to become slidable thereon.

As a result, a gap is formed between the concavely curved surface and the sheet opposed to the concavely curved surface in pristine condition, thus leading to the likelihood of causing tongue coating to accumulate in the gap.

For this reason, one may consider, for example, bending the sheet beforehand so as to permit the same to go along in parallel with the concavely curved surface so that no, or the slightest possible gap, if any, may be formed between the concavely curved surface and the sheet opposed to the concavely curved surface, which, however, would result in not only the increase of the manufacturing steps but the difficulties in forming the sheet so as to allow the same to go precisely along the concavely curved surface.

When removing the grime in tongue coating in such a way as were disclosed in the patent documents 3, 4, a loop is allowed to contact with a tongue and then move along a surface of the tongue, thus scraping out the grime hidden in the tongue coating.

Whereas, there exist a number of minute mucosal protrusions, called tongue papillae, on a rear side, sides, etc. of a tongue. These tongue papillae are, e. g., on the order of 0.5 to 1mm in size.

According to the conventional tongue cleaners, when scraping out the grime attached to tongue papillae with the loop, there is a possibility of injuring the tongue papillae with the loop.

Therefore, a problem to be solved by the first aspect, not forming part of the present invention, is to form, from the beginning, no or the slightest possible gap between the concavely curved surface formed in a head and the sheet covering the concavely curved surface, thereby making it less likely for tongue coating to accumulate in the gap. Further, another problem to be solved by the present disclosure is to manufacture a tongue cleaner either without such gap or with the slightest possible one.

Further, with respect to a tongue cleaner provided with a plurality of filiform materials for the sake of scraping out tongue coating, the present invention provides a tongue cleaner enabling resultant scratches on a tongue as few as possible.

A first aspect not forming part of the invention is a tongue cleaner including:
a stick-shaped handle; a flat-and-thin head provided at a distal end of said stick-shaped handle; and a sheet attached to a face of said head, said sheet having a surface roughened,
wherein said sheet is formed either in close contact with said face or with a slight gap between said sheet and said face.

A second aspect not forming part of the invention is a tongue cleaner including: a stick-shaped handle; a flat-and-thin head provided at a distal end of said stick-shaped handle, said flat-and-thin head including, on either a front or reverse face thereof, a concavely curved surface with a valley portion extending parallel to a longitudinal direction of said stick-shaped handle; and a first sheet attached to said concavely curved surface, said first sheet having a surface roughened and being fixed on a lateral side of said head,
wherein said first sheet is formed into a curved surface such that said first sheet is attached either in close contact with said concavely curved surface or with a slight gap between said sheet and said concavely curved surface.

A third aspect not forming part of the invention is a method for manufacturing a tongue cleaner including a stick-shaped handle; a flat-and-thin head provided at a distal end of said stick-shaped handle; and a sheet attached to a face of said head, said sheet having a surface roughened, the method comprising:
pressing a material for said sheet against said face so that said sheet is attached either in close contact with said face or with a slight gap between said sheet and said face.

A fourth aspect not forming part of the invention is a method for manufacturing a tongue cleaner including a stick-shaped handle; a flat-and-thin head provided at a distal end of said stick-shaped handle, said flat-and-thin head having, on either a front or reverse face thereof, a concavely curved surface with a valley portion extending parallel to a longitudinal direction of said stick-shaped handle; and a first sheet attached to said concavely curved surface, said first sheet having a surface roughened and being fixed on a lateral side of said head, the method comprising:
pressing a material for said first sheet against said face to form said first sheet into a curved surface such that said first sheet is attached either in close contact with said concavely curved surface or with a slight gap between said sheet and said concavely curved surface.

A fifth aspect not forming part of the invention is a method for manufacturing a tongue cleaner including a stick-shaped handle; a flat-and-thin head provided at a distal end of said stick-shaped handle, said flat-and-thin head including, on front and reverse faces thereof, a concavely curved surface and a convexly curved surface having a valley portion and a raised portion, respectively, each extending parallel to a longitudinal direction of said stick-shaped handle; a first sheet attached to said concavely curved surface, said first sheet having a surface roughened and being fixed to a lateral side of said head; and a second sheet attached to said convexly curved surface, said second sheet having a surface roughened and being fixed to a lateral side of said head, the method comprising:
pressing a material for said first sheet against said concavely curved surface to form said first sheet into a curved surface such that said first sheet is attached either in close contact with said concavely curved surface or with a slight gap between said sheet and said concavely curved surface; and
pressing a material for said second sheet against said convexly curved surface to form said second sheet into a curved surface such that said second sheet is attached either in close contact with said convexly curved surface or with a slight gap between said second sheet and said convexly curved surface.

A sixth aspect not forming part of the invention is a method for manufacturing a tongue cleaner including a stick-shaped handle; a flat-and-thin head provided at a distal end of said stick-shaped handle, said flat-and-thin head including, on either a front or reverse face thereof, a concavely curved surface with a valley portion extending parallel to a longitudinal direction of said stick-shaped handle; and a first sheet attached to said concavely curved surface, said first sheet having a surface roughened and being fixed to a lateral side of said head, the method comprising:
arranging said first sheet on said concavely curved surface with said head being in a single piece;
pressing a material for said first sheet against said concavely curved surface to form said first sheet into a curved surface such that said first sheet is attached either in close contact with said concavely curved surface or with a slight gap between said first sheet and said concavely curved surface; and
providing a ring integral with said stick-shaped handle on a lateral side of said head that has now been integrated with said first sheet to thereby fix said first sheet on the lateral side of said head.

A seventh aspect not forming part of the invention is a method for manufacturing a tongue cleaner including a stick-shaped handle; a flat-and-thin head provided at a distal end of said stick-shaped handle, said flat-and-thin head including, on front and reverse faces thereof, a concavely curved surface and a convexly curved surface having a valley portion and a raised portion, respectively, each extending parallel to a longitudinal direction of said stick-shaped handle; a first sheet attached to said concavely curved surface, said first sheet having a surface roughened and being fixed to a lateral side of said head; and a second sheet attached to said convexly curved surface, said second sheet having a surface roughened and being fixed to a lateral side of said head, the method comprising:
arranging said first sheet on said concavely curved surface, while arranging said second sheet on said convexly curved surface with said head being in a single piece;
pressing a material for said first sheet against said concavely curved surface to form said first sheet into a curved surface such that said first sheet is attached either in close contact with said concavely curved surface or with a slight gap between said first sheet and said concavely curved surface;
pressing a material for said second sheet against said convexly curved surface to form said second sheet into a curved surface such that said second sheet is attached either in close contact with said convexly curved surface or with a slight gap between said second sheet and said convexly curved surface;
providing a ring integral with said stick-shaped handle on a lateral side of said head that has now been integrated with said first and second sheets to thereby fix said first and second sheets on the lateral side of said head.

An eighth aspect not forming part of the invention is a method for manufacturing a tongue cleaner according to any one of the first to seventh aspects in which said sheet(s) is/are a pile-woven textile.

A ninth aspect not forming part of the invention is a method for manufacturing said tongue cleaner according to the eighth aspect, the method further comprising:
processing said textile and thus forming depressed portions and protruded portions along the longitudinal direction of the textile to form the longitudinal direction of the textile into a corrugated shape.

A tenth aspect not forming part of the invention is a method for manufacturing said tongue cleaner according to the eighth aspect, the method furthermore comprising:
a disconnecting part in the longitudinal direction of said textile.

An eleventh aspect of the present invention is a tongue cleaner according to independent claim 1, with preferred embodiments disclosed in the dependent claims. The tongue cleaner including a plurality of protruding looped linear materials on a main body of said tongue cleaner, wherein each of said linear materials is provided with a bridging portion between distal ends of first and second protrusions located at intervals,
wherein at least parts of said first and second protrusions and at least said bridging portion are provided with depressed and protruded portions along the longitudinal direction of each of said linear material to form the longitudinal direction of each of said linear material into a corrugated shape.

A twelfth aspect of the present invention is the tongue cleaner according to the eleventh aspect, wherein an approximately central portion in the longitudinal direction of said bridging portion is formed into an circular-arch shape protruding upward.

A thirteenth aspect of the present invention is the tongue cleaner according to the eleventh aspect or the twelfth aspect, wherein said first and second protrusions and said bridging portion are provided with depressed and protruded portions along the longitudinal direction of each of said linear material to form the longitudinal direction of each of said linear material into a corrugated shape.

A fourteenth aspect of the present invention is the tongue cleaner according to any one of the eleventh to fourteenth aspects, wherein said linear material is loop pile.

A fifteenth aspect not forming part of the invention is a tongue cleaner including a plurality of protruding linear materials on a main body of said tongue cleaner, wherein at a distal end of said protrusion, each of said linear material is provided with a longitudinal part of a rising portion to a direction intersecting with the longitudinal direction of said protrusion and besides the distal end of said rising portion is formed into a free end.

A sixteenth aspect not forming part of the is the tongue cleaner according to the fifteenth aspect, wherein the longitudinal direction of said rising portion is provided sideways in relation to the longitudinal direction of said protrusion.

A seventeenth aspect not forming part of the invention is the tongue cleaner according to the fifteenth aspect or sixteenth aspect, wherein the distal end of said rising portion is folded toward the main body of said tongue cleaner.

A eighteenth aspect not forming part of the invention is the tongue cleaner according to any one of the fifteenth to seventeenth aspects, wherein an approximately central portion in the longitudinal direction of said rising portion is formed into a circular-arc shape protruding upward.

According to the first aspect not forming part of the invention, the sheet is allowed to be formed in close contact with the face of the head, or otherwise, with the slight gap therebetween. Hence, the sheet is made slightly slidable on the face of the head and therefore the tongue coating scraped out into the portion between the surface and the sheet becomes less likely to accumulate in the gap thus made slight, so that a good sanitary condition can be kept.

According to the second aspect not forming part of the invention, the first sheet is allowed to be formed either in close contact with the concavely curved surface or with the slight gap therebetween. Hence, the first sheet is made slightly slidable on the concavely curved face of the head and further the tongue coating scraped out into the portion between the surface and the sheet becomes less likely to accumulate in the gap made slight, so that a good sanitary condition can be kept.

According to the third aspect not forming part of the invention, the sheet is made slightly slidable on the face of the head and the material for the sheet is allowed to be pressed against the face of the head to be in close contact with the face of the head or with the slight gap therebetween. Hence, the tongue coating scraped out into the portion between the surface and the first sheet becomes less likely to accumulate in the gap thus made slight, so that a good sanitary condition can be held.

According to the fourth aspect not forming part of the invention, the first sheet is made slightly slidable on the concavely curved face of the head, and the material for the first sheet is pressed against the concavely curved surface to form the first sheet into such a curved surface that the first sheet is attached in close contact with the concavely curved surface or with the slight gap therebetween. Hence, the tongue coating scraped out into the portion between the concavely curved surface and the first sheet becomes less likely to accumulate in the gap thus made slight, so that a good sanitary condition can be kept.

According to the fifth aspect not forming part of the invention, the first sheet and the second sheet are made slightly slidable on the curved faces of the head, and the material for the first sheet is pressed against the concavely curved surface to form the first sheet into such a curved surface that the first sheet is attached in close contact with the concavely curved surface or with the slight gap formed therebetween. Hence, the tongue coating scraped out into the portion between the concavely curved surface and the first sheet becomes less likely to accumulate in the gap thus made slight, so that a good sanitary condition can be kept.

According to the sixth aspect not forming part of the invention, the material for the first sheet is arranged on the concavely curved surface with the head being in a single-piece state to press the first sheet against the concavely curved surface and besides the ring is provided on the lateral side of the head. Hence, the first sheet can be fitted precisely and slidably on the concavely curved face of the head being in a single-piece state so as to be attached in close contact with the concavely curved face of the head or with the slight gap therebetween.

According to the seventh aspect not forming part of the invention, with the head being in a single-piece state, the materials for the first and second sheets are arranged on the concavely and convexly curved surfaces, respectively, and the materials for the first and second sheets are pressed against the concavely and convexly curved surfaces, respectively, and the ring is provided on the lateral side of the head. Hence, the first and second sheets can be fitted precisely and slidably on the concavely and convexly curved surfaces, respectively, of the head being in a single-piece state so as to be attached in close contact with the concavely and convexly curved surfaces of the head, respectively, or with the slight gap therebetween.

According to the eighth aspect not forming part of the invention, the pile-woven textile enables tongue coating to be scraped out.

According to the ninth aspect not forming part of the invention, the depressed portions and the protruded portions can be easily provided on the textile. Hence, when the textile catches tongue papillae, the depressed portions and the protruded portions linearly stretch and thus each of the linear material can slip through the tongue papillae.

According to the tenth aspect not forming part of the invention, the disconnected portions can be easily provided in the textile. Hence, when the textile catches tongue papillae, the disconnected portions expand to cause the textile to stretch and thus each of the linear material can slip through the tongue papillae.

According to the eleventh aspect of the present invention, when the textile catches tongue papillae, the depressed portions and the protruded portions linearly stretch and thus each of the linear material can slip through the tongue papillae.

According to the twelfth aspect of the present invention, when tongue papillae are located on the edge of the bridging portion, if each of the linear material is moving relatively to the tongue, the tongue papillae can relatively move to the approximately central portion of the bridging portion and thus each of the linear material can easily slip through the tongue papillae.

According to the thirteenth aspect of the present invention, the rate at which the depressed portions and protruded portions become linear for each of the linear materials to stretch can be increased as far as possible.

According to the fourteenth aspect of the present invention, the tongue cleaner can be pressed against the surface of a tongue with appropriate elasticity.

According to the fifteenth aspect not forming part of the invention, the rising portion is pulled by tongue papillae for the rising portion to deform along the longitudinal direction of the protrusion and thus the rising portion can slip through the tongue papillae.

According to the sixteenth aspect not forming part of the invention, the rising portion becomes sideways to the moving direction. Hence, the grime of tongue coating can be surely rubbed off without failing to do so.

According to the seventeenth aspect not forming part of the invention, the protrusion, the rising portion and the distal end of the folded portion envelop tongue coating. Hence, the grime of tongue coating can be more surely rubbed off.

According to the eighteenth aspect not forming part of the invention, when tongue papillae touch the rising portion, the circular-arc-shaped rising portion can move along so as to slide on the tongue papillae and thus each of the linear material can slip through the tongue papillae.
FIG. 1 is a perspective view illustrating a tongue cleaner according to a first embodiment not forming part of the invention.
FIG. 2 is a cross-sectional view on a line A-A in FIG. 1.
FIG. 3 is a cross-sectional view on a line B-B in FIG. 1.
FIG. 4 is a cross-sectional view illustrating a main section according to the first embodiment not forming part of the invention.
FIG. 5 is a cross-sectional view illustrating the tongue cleaner in actual use according to the first embodiment not forming part of the invention.
FIG. 6 is a cross-sectional view illustrating a first step of manufacturing a head of the first embodiment not forming part of the invention.
FIG. 7 is a cross-sectional view illustrating a second step of manufacturing the above head.
FIG. 8 is a plan view illustrating a mold in which the above head is set.
FIG. 9 is a vertical cross-sectional view illustrating the mold in which the above head is set.
FIG. 10 is a cross-sectional view illustrating a first step of manufacturing a head according to a second embodiment not forming part of the invention.
FIG. 11 is a cross-sectional view a second step of manufacturing the above head.
FIG. 12 is a vertical cross-sectional view illustrating a mold in which the above head is set.
FIG. 13 is a perspective view illustrating a tongue cleaner according to a third embodiment not forming part of the invention.
FIG. 14 is an enlarged vertical cross-sectional view illustrating a main section according to a fourth embodiment of the present invention.
FIG. 15 is an enlarged plan view illustrating a usage condition according to the fourth embodiment of the present invention.
FIG. 16 is an enlarged vertical cross-sectional view illustrating the main section according to a fifth embodiment not forming part of the invention.
FIG. 17 is an enlarged plan view illustrating a usage condition according to the fifth embodiment not forming part of the invention.
FIG. 18 is an enlarged vertical cross-sectional view illustrating a main section according to a sixth embodiment not forming part of the invention.
FIG. 19 is an enlarged perspective view illustrating a partially cut out section according to a seventh embodiment not forming part of the invention.

Preferred embodiments of the present invention are described with reference to the accompanying drawings. It should be noted that the embodiments described below are not to limit the scope of the present invention set forth in the claims. Further, all the schemes described below are not necessarily the requirements of the present invention.

### First embodiment not forming part of the present invention

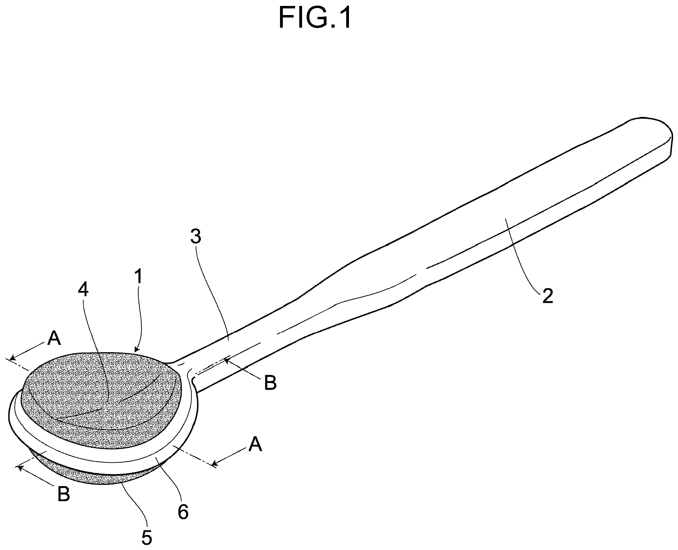

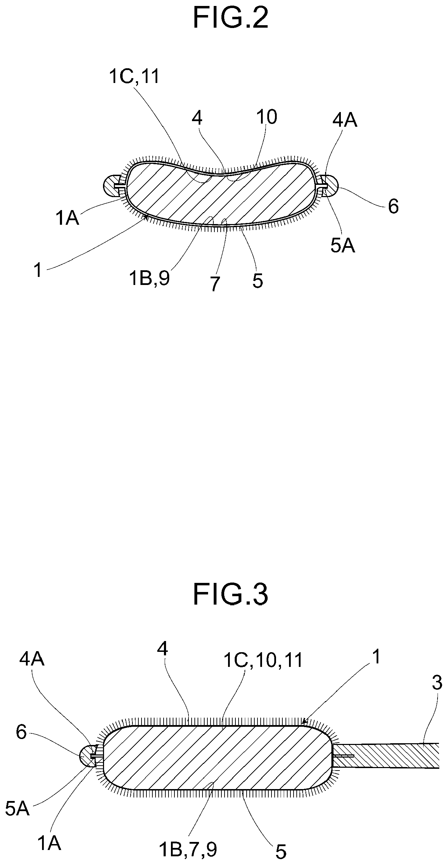

As shown in FIG. 1 to FIG. 5, a tongue cleaner includes an substantially straight stick-shaped handle 2 in a head 1 forming a main body of a cleaner. The head 1 and the stick-shaped handle 2 are connected with each other via a thin and slightly flexible neck 3. The head 1 is formed in a flat-and-thin shape and a face of the head 1 is covered with first and second sheets 4, 5 each having a surface roughened. The head 1 includes a ring 6 which is fixed to an outside of a lateral side 1A of the head 1 via the sheets 4, 5 and is connected to an end of a neck 3. The head 1 has a flattened and substantially isosceles triangle shape or equilateral triangle shape in plan view such that it is widened at its distal end and narrowed at its neck 3 side. In the present embodiment, the head 1 is formed in a substantially equilateral triangle shape whose corners are rounded. The head 1 has a first surface on a flat portion 1B side, e.g., an underside in FIG. 2, formed into such a convexly curved surface 9 that a raised portion 7 extends along a longitudinal direction of the stick-shaped handle 2 so as to fit the surface of a tongue 8. On the other hand, the head 1 has a second surface on a flat portion 1C side, e.g., an upper side in FIG. 2, formed into such a concavely curved surface 11 that a valley portion 10 extends along the longitudinal direction of the stick-shaped handle 2 so as to fit the surface of the tongue 8.

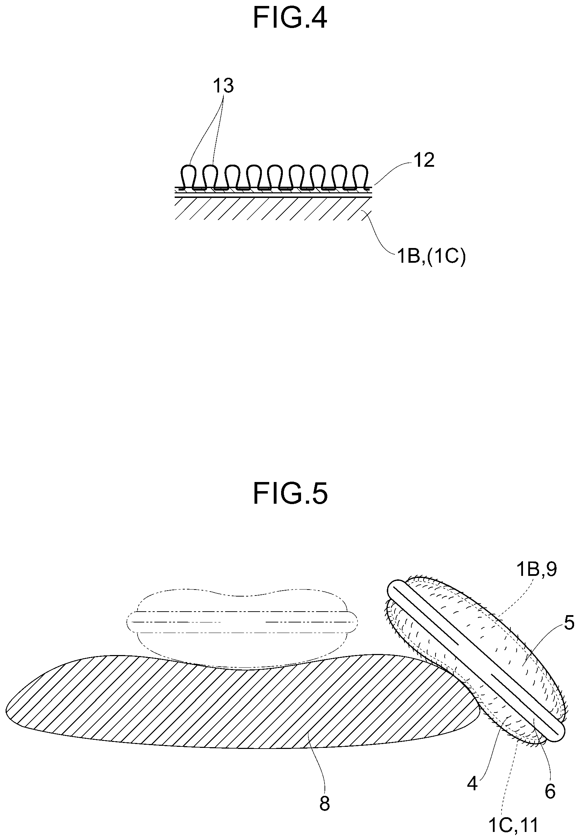

Then, the above concavely curved sheet 4 having a concavely curved vertical cross section is provided such that it is attached substantially in close contact with the concavely curved surface 11 or with a slight gap between the sheet 4 and the concavely curved surface 11. Further, the convexly curved sheet 5 having a convexly curved vertical cross section is provided such that it is attached in close contact with the convexly curved surface 9. As shown in FIG. 4, the sheets 4, 5 are provided with fibers 13 on the surface of a basal main body 12 thereof in a manner protruding outward toward the top and bottom, respectively. According to the present embodiment, a pile-woven textile is used such that the sheet 5 is provided so as to cover one flat portion 1B on the head 1 and a first half vertical portion on the lateral side 1A, while the sheet 4 is provided so as to cover the other flat portion 1C on the head 1 and a second half vertical portion on the lateral side 1A. Then, the edge 4A of the sheet 4 and the edge 5A of the sheet 5 are mated with each other by fitting the ring 6 on the lateral side 1A at the distal, lateral and neck 3 sides of the head 1 to thereby integrate the head 1, the edge 4A of the sheet 4, the edge 5A of the sheet 5 and the ring 6 with one another. It should be noted that the sheets 4, 5 may be formed from woven sheets including twill-woven textile, sateen-woven textile, etc., unwoven textile, or a surface-roughened material such as a porous material or a multi-void material, other than from a pile-woven textile.

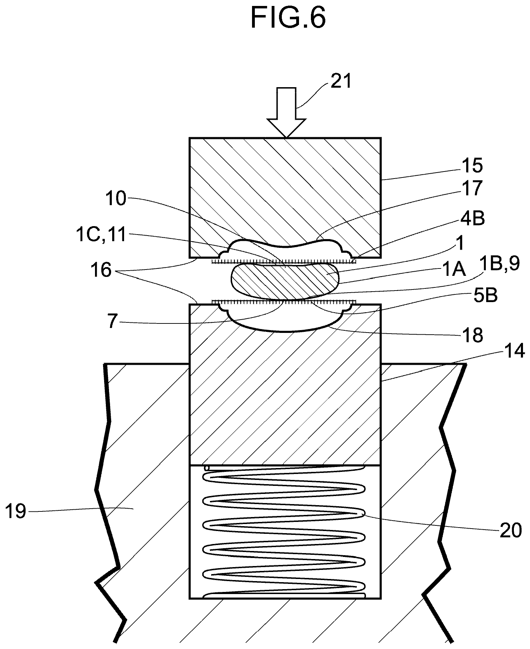

Next is a description of a method for manufacturing the tongue cleaner. In manufacturing the head 1, as shown in FIG. 6, there are provided a pair of a lower mold 14 and an upper mold 15 opposed to the lower mold 14 so as to be unitable with and separable from the lower mold 14. A concavely curved surface molding convex portion 17 with the same shape as that of the concavely curved surface 11 is formed on the underside of the upper mold 15, being one of the pair of divided surfaces 16, while a convexly curved surface molding concave portion 18 with the same shape as that of the convexly curved surface 9 is formed on the upper side of the lower mold 14, being the other of the pair of divided surfaces 16. Here, the lower mold 14 is supported on a pedestal 19 via an elastic means 20 such as an upwardly-urging spring or the like, while the upper mold 15 is provided with a pressing means 21 such as a press mechanism, etc.

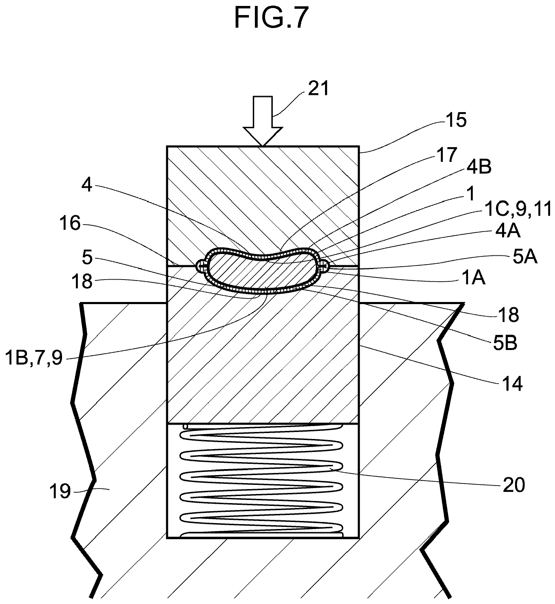

Then, a sheet material 5B made of a flat pile-woven textile for forming the sheet 5 and having its fibers 13 directed downwards, the head 1 having the convexly curved surface 9 directed downwards, and a sheet material 4B made of a flat pile-woven textile for forming the concavely curved sheet 4 and having its fibers directed upward are sequentially stacked, and thereafter the pressing means 21 is activated to press the upper mold 15 to the lower molds 14, whereby the sheet material 5B, the head 1 and the sheet material 4B are, as shown in FIG. 7, sandwiched, with pressure load being applied thereto, between the convexly curved surface molding concave portion 18 and the concavely curved surface molding convex portion 17. As a result, the sheet material 4B is sandwiched between the concavely curved surface molding convex portion 17 and the concavely curved surface 11 to be formed into a curved shape so as to form the valley portion extending in the longitudinal direction along the concavely curved surface 11 to thereby form the concavely curved sheet 4. At the same time, the sheet material 5B is sandwiched between the convexly curved surface molding concave portion 18 and the convexly curved surface 9 to be formed into a curved shape so as to form the raised portion extending in the longitudinal direction along the convexly curved surface 9 to thereby form the convexly curved sheet 5. Further, the respective edges 4A, 5A of the sheets 4, 5 are allowed to be coupled to each other on the lateral side 1A of the head 1 and the outside portions of the coupled edges are cut off.

Then, the mold is opened to separate the divided surfaces 16 from each other and then the integrated piece comprising the head 1, the concavely curved sheet 4 and the concavely curved sheet 5 is taken out. In this head 1, the sheet 4 molded into a concavely curved shape is either in close contact with the concavely curved surface 11 or with a slight gap formed therebetween and therefore the sheet 4 is not fixed to the concavely curved surface 11 so as to be slightly slidable on the concavely curved surface 11. Likewise, the sheet 5 molded into a convexly curved surface is either in close contact with the concavely curved surface 11 or with a slight gap formed therebetween and therefore the sheet 5 is not fixed to the convexly curved surface 9 so as to be slightly slidable on the convexly curved surface 9.

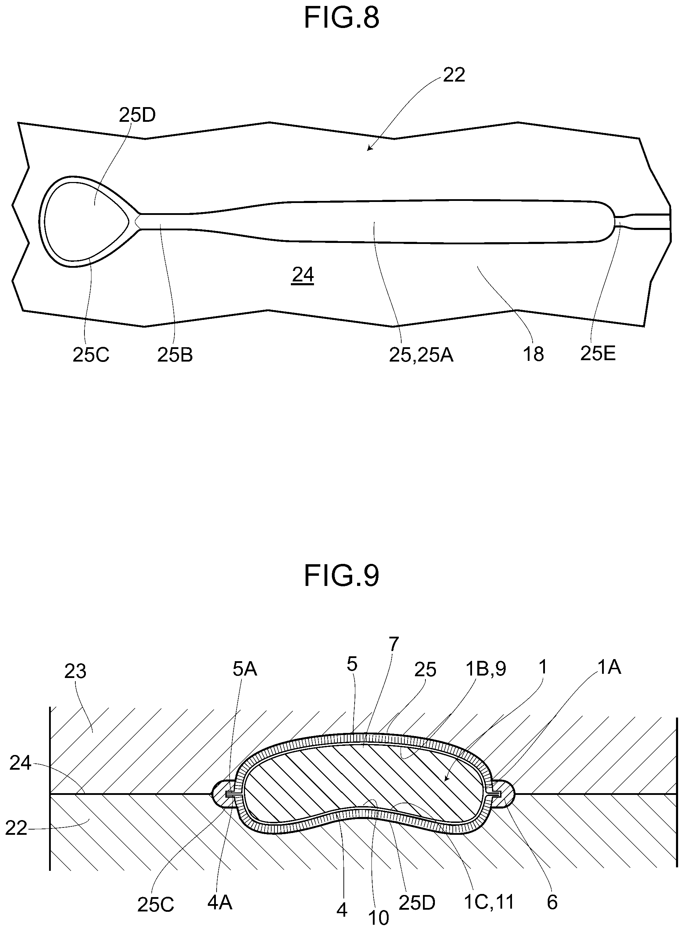

Next, the stick-shaped handle 2 and the ring 6 are molded using an injection mold shown in FIG. 8 and FIG. 9. The molding performed using this injection mold is to mold the stick-shaped handle 2, the neck 3, and the ring 6 integrally with the head 1 that has been integrated with the sheets 4, 5, with such head 1 being nested thereinside. Accordingly, the injection mold includes a lower mold 22 and an upper mold 23 which are unitable with and separable from each other, and a cavity 25, acting as a molding space formed in a halved manner on the respective divided surfaces 24 of the upper and lower molds 24, 25, comprises a ring molding section 25C formed at a frontal portion of a stick-shaped handle molding section 25A via a neck molding section 25B. Further, a placing section 25D for the preformed head 1 integrated with the sheets 4, 5 is formed inside this ring molding section 25C. This placing section 25D is formed in the same shape as the concavely curved surface 11 so that the sheet 4, which has already been integrated with the head 1 and formed in a concavely curved shape, can engage therewith. In addition, the cavity 25 of the upper mold 15 opposed to the placing portion 25D is formed in the same shape as the convexly curved surface 9. Furthermore, a feed opening 25E for feeding molten resin is formed at an end of the stick-shaped handle molding section 25A.

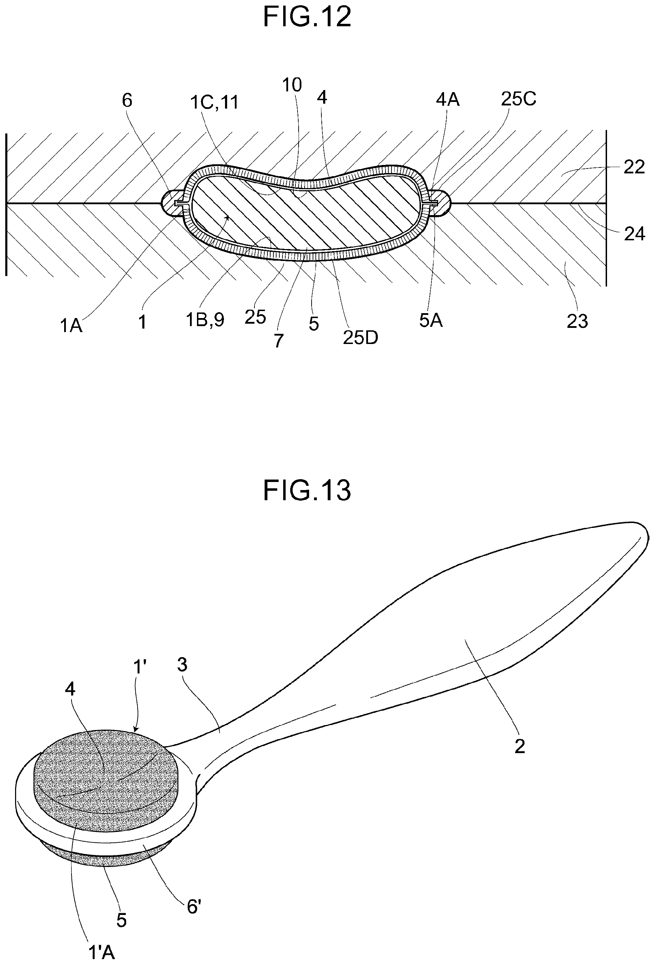

Accordingly, with the injection mold being opened, the head 1 is placed on the placing section 25D on an upper surface of the lower mold 22 with the sheet 4 placed at the underside and the sheet 5 placed at the upper side and then the injection mold is closed. Then, the molten resin fed from the feed opening 25E is allowed to fill the stick-shaped handle molding section 25A, the neck molding section 25B and the ring molding section 25C. At this moment, the resin filled in the ring molding section 25C comes in contact with the lateral side 1A of the head 1 and thus the ring 6 is allowed to be fitted to an outside of the lateral side 1A of the head via the edges 4A, 5A of the sheets 4, 5, thus permitting the head 1 to be fixed to an inside of the ring 6. Alternatively, as shown in FIG. 12 referred to hereinafter, the head 1 may be placed on the placing portion 25D with the sheet 4 placed at the upper side and the sheet 5 placed at the downside.

Then, after subjecting the molding to cooling, the mold is opened to take out a product. It should be noted herein that in the product thus manufactured, the concavely curved sheet 4 molded in a concavely curved shape is either in close contact with the concavely curved surface 11 or with a slight gap formed therebetween, while the sheet 4 is not fixed to the concavely curved surface 11 so that it is made slightly slidable on the concavely curved surface 11. Similarly, the convexly curved sheet 5 molded in a convexly curved shape is either in close contact with the convexly curved sheet 5 or with a slight gap formed therebetween, whilst the convexly curved sheet 5 is not fixed to the convexly curved surface 9 so that it is made slightly slidable on the convexly curved surface 9.

Consequently, as shown in FIG. 5, when grasping the stick-shaped handle 2 to let the head 1 enter a mouth and apply the head 1 to a convex portion of a tongue 8 such as a corner thereof, the head 1 is allowed to contact with the tongue 8 with the sheet 4, attached to the concavely curved surface 11, being faced downwards, thus scraping out tongue coating. In this case, the sheet 4 provided outside relative to the concavely curved surface 11 of the head 1 is slightly slidable without a gap and hence even if somewhat a large force is applied to the stick-shaped handle 2, such force is not transmitted at once from the head 1 to the sheet 4 but it is relaxed due to the sliding motion of the sheet 4, thus enabling the likelihood of injuring the tongue 8 to be reduced. Further, the sheet 4 is slidably attached in close contact with the concavely curved surface 11 in advance in a manner extending along the concavely curved surface 11 and hence there is no gap between the sheet 4 and the concavely curved surface 11, enabling the likelihood of the accumulation of tongue coating to be reduced.

Further, as shown with chain double-dashed lines in FIG. 5, the sheet 5 on the convexly curved surface 9 is applied to a place where the concavity of the tongue 8 exists. In that case, the convexly curved sheet 5 provided on the outside of the convexly curved surface 9 of the head 1 is able to be slightly slidable thereon, and therefore even if somewhat large force is applied to the stick-shaped handle 2, such force is not transmitted at once from the head 1 to the sheet 5 such that it is buffered due to the sliding motion of the sheet 5, thus permitting the likelihood of injuring the tongue 8 to be reduced.

Then, after use, the head 1 is washed with water and then it is dried.

As described above, according to the present embodiment, the first sheet 4 is provided on the face of the head 1 formed with the concavely curved surface 11 and besides it is fixed to the lateral side 1A of the head 1 to thereby enable the sheet 4 opposed to the concavely curved surface 11 to slide. Furthermore, the second sheet 5 is provided on the face of the head 1 formed with the convexly curved surface 9 and besides it is fixed to the lateral side 1A of the head 1 to thereby enable the sheet 5 opposed to the convexly curved surface 9 to slide. Therefore, the sheets 4, 5 are allowed to come in soft contact with the tongue 8, and even if a strong force is applied to the stick-shaped handle 2, it can be buffered by the sliding motions of the sheets 4, 5. Furthermore, since the sheet 4 is formed so as to be attached in close contact with the concavely curved surface 11 and thus the sheet 4 and the concavely curved surface 11 are allowed to be attached either in close contact with each other or with a slight gap therebetween without forming a large gap, the tongue coating scraped out becomes less likely to accumulate in the gap therebetween, thus permitting a good sanitary condition to be maintained.

Moreover, according to the foregoing method for manufacturing the tongue cleaner, the sheet material 4B of the sheet 4 arranged on the concavely curved surface 11 is pressed against the concavely curved surface 11 and thus the sheet material 4B is allowed to be in close contact with the concavely curved surface 11 or with a slight gap formed therebetween. Hence, the sheet 4 can be precisely formed. At the same time, the sheet material 5B of the sheet 5 arranged on the convexly curved surface 9 is pressed against the convexly curved surface 9 by the upper and lower molds 14, 15 and thus the sheet 5 is allowed to be in close contact with the convexly curved surface 9 or with a slight gap formed therebetween. Hence, in similar fashion to the sheet 4, the sheet 5 can be precisely formed, as well.

Yet more, after arranging the sheet material 4B on the concavely curved surface 11 with the head 1 being in a single-piece state as a component of the tongue cleaner, the sheet material 4B of the sheet 4 is pressed against the concavely curved surface 11 to form the sheet 4 into the curved surface in close contact with the concavely curved surface 11. Then, the ring 6 integrated with the stick-shaped handle 2 is provided on the lateral side 1A of the head 1 to fix the sheet 4 on the lateral side 1A of the head 1. As a result, the sheet 4 can be fitted so as to be precisely attached in close contact with the concavely curved surface 11 of the head 1 being in such a single-piece state or with a slight gap formed therebetween. Further, after arranging the sheet material 5B of the sheet 5 on the convexly curved surface 9 with the head 1 being in a single-piece state, the sheet material 5B of the sheet 5 is pressed against the convexly curved surface 9 by means of the upper and lower molds 14, 15 and thus the sheet 5 can be formed so as to be precisely attached in close contact with the convexly curved surface 9 of the head 1 being in a single-piece state or with a slight gap formed therebetween.

### Second embodiment not forming part of the present invention

Hereunder is a description of other embodiments, in which the same numerical symbols are attached to the same parts as those of the first embodiment and their detailed descriptions are omitted.

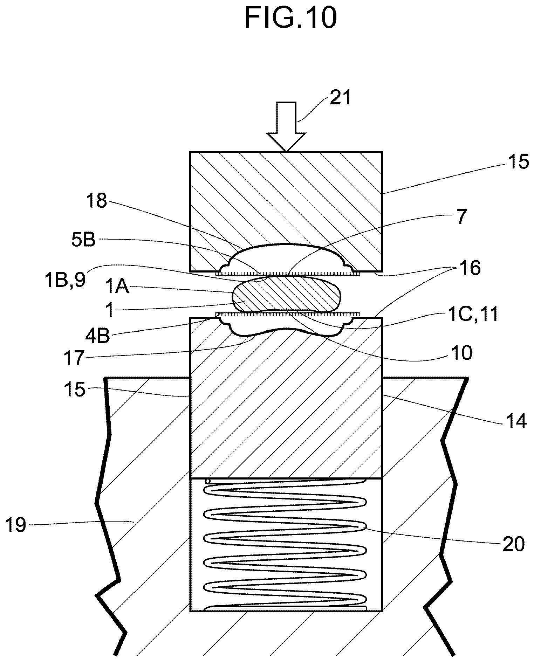

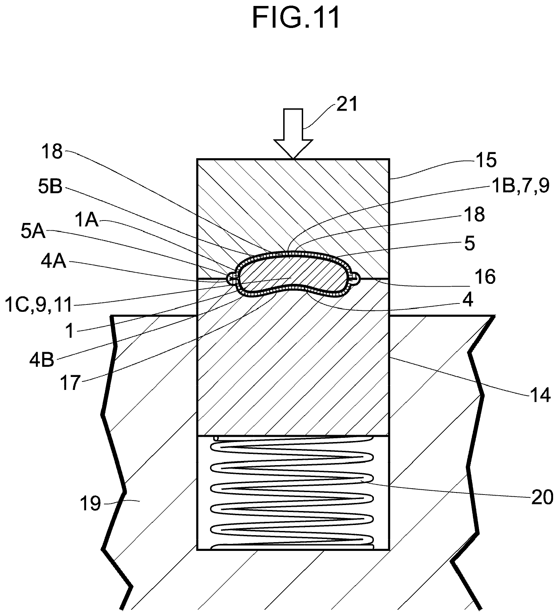

In a second embodiment shown in FIG. 10 to FIG. 12, unlike the first embodiment, the concavely curved surface molding convex portion 17 is formed on the lower mold 14 and the convexly curved surface molding concave portion 18 is formed on the upper mold 15. Further, as for the injection mold, the placing section 25D of the lower mold 22 is formed in the same shape as the convexly curved surface 9, and the placing section 25D of the upper mold 23 is formed in the same shape as the concavely curved surface 11.

Then, the sheet material 4B made of a flat pile-woven textile for forming the concavely curved sheet 4 and having its fibers 13 faced downwards, the head 1 with its convexly curved surface 9 faced upwards, and the sheet material 5B with its fibers 13 faced upwards are sequentially stacked. Thereafter, the pressing means 21 is activated to press the upper and lower molds 15, 14 and as a result, the integrated head is produced in the same manner as in the first embodiment with the sheets 4, 5 allowed to be in close contact with the head 1 or with a slight gap formed between the sheets 4, 5 and the head 1.

Further, the head 1 with the sheet 5 faced downwards and the sheet 4 faced upwards is placed on the placing section 25D and then the injection mold is closed to mold the stick-shaped handle 2, the neck 3 and the ring 6. In addition, as shown in FIG. 9, the concavely curved surface 11 may be placed on the placing section 25D to perform molding.

### Third embodiment not forming part of the present invention

FIG. 13 shows a third embodiment, in which the same numerical symbols are attached to the same parts as those of the first embodiment and their detailed descriptions are omitted.

In the third embodiment, a plane of the head 1' is formed in a circular shape. The ring 6' is arranged on a lateral side 1'A of this circular head 1'. In the third embodiment as well, the same operational effect as that obtained in the first embodiment can be attained.

### Fourth embodiment according to the present invention

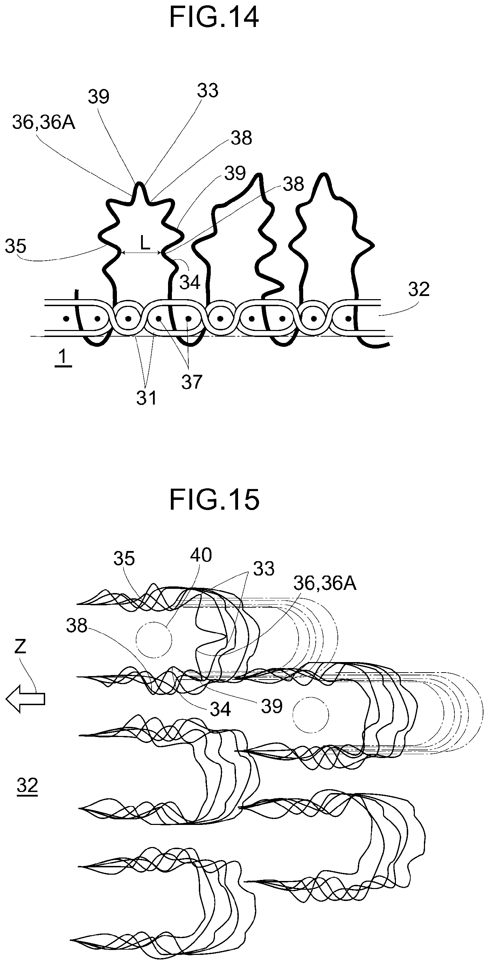

As shown in FIG. 14 and FIG. 15, in the fourth embodiment, loop-pile-woven textile is used which is made of mutifilament yarns formed by overlapping a plurality of filaments. FIG. 14 is a cross-sectional view of the pile-woven textile. In the pile-woven textile, it is woven such that in part (a pile yarn 31) of warps (pile yarns 31, a base warp), a ring-like loop 33 is woven in a protruding fashion as a linear material on the surface of the pile-woven base textile 32 and therefore the pile yarn loop 33 is allowed to catch tongue coating to make the tongue coating easy to be raised, making it possible to more effectively scrape out the grime of the tongue coating. The innumerable loops 33 made of pile yarns, protruding in a ring-like manner on a surface of this pile-woven base textile 32 are attached to the head 1 in a direction intersecting with (desirably perpendicular to) a plane surface of the head 1. Then, the head 1 pressed against a tongue is allowed to move back and forth in the longitudinal direction of the head 1, defined as a moving direction Z, and thus the innumerable loops 33 made of the pile yarns, being warps rising from the surface of the pile-woven base textile 12 get entangled with the tongue coating to raise the tongue coating, making it easy to scrape out the grime. As such, the loop 33 made of the pile yarn includes a right and left pair of or a front and rear pair of protrusions 34, 35 which stand at an interval L and rise from the base textile 32; and a bridging portion 36 which bridges distal ends of the pair of the upright protrusions 34, 35. Note that numeral symbol 37 denotes a weft. In addition, the direction of the interval L between the protrusions 34, 35 intersects with the moving direction Z, which make a right angle in the present embodiment.

Further, the loop 33 includes a repeated pattern of a depressed portion 38 and a protruded portion 39 along the longitudinal direction of the loop 33 to form the longitudinal portion of the loop 33 into a corrugated shape. Alternatively, these depressed and protruded portions may be formed not along the entire length but only partially therealong.

Accordingly, when the head 1 is allowed to enter a mouth with the stick-like handle held by hand to apply the head 1 to the depressed portion of a tongue, the loop 33 is allowed to contact with the tongue, and thus the loop 33 falls down along the surface of the tongue to scrape out the grime of tongue coating. On the other hand, when the head 1 is allowed to enter the mouth with the stick-like handle held by hand to apply the head 1 to the protruded position of the corner or the like of the tongue, the head is allowed to contact with the tongue with the concavely-curved surface faced downwards to rub off the grime of the tongue coating in such a way as to scrape out the grime by the loop 33 made of the pile yarn 31.

When the loop 33 is moved in the moving direction Z to scrape out the grime of the tongue coating by the loop 33 in this way, tongue papillae 40, for example, may thrust into the loop 33 and the bridging portion 36 of the loop 33 may be caught by the tongue papillae 40. In that case, as shown by the dashed-dotted lines in Fig.15, the corrugated portions of the depressed portion 38 and the protruded portion 39 in the loop 33 are allowed to extend linearly to act as a cushion and then the bridging portion 36 of the loop 33 is allowed to slide relative to the tongue papillae 40, thus permitting the bridging portion to slip through the tongue papillae 40 during this sliding motion. This slipping-through motion can reduce the possibility of injuring the tongue papillae 40 by the loop 33.

As for a manufacturing method for forming the depressed portion 38 and the protruded portion 39 along the longitudinal direction of the loop 33, it is performed in the following manner. A rotating brush (not shown), for example, is pressed against the looped textile 13 in an unprocessed state prior to being mounted on the head 1 as shown in FIG. 4 and then it is subjected to plastic processing to form depressed and protruded portions on the textile 13. Specifically, the pressure from the rotating brush or the like is applied to the textile 13 to deform the textile 13 over the limit of elasticity so that even after the pressure is removed, the deformation is left unchanged. The one processed thus way is attached to the head 1.

As described above, according to the fourth embodiment, there is provided the tongue cleaner in which a plurality of the loops is arranged on the head 1 as a linear material. The loop 33 is provided with the bridging portion 36 on the distal ends of the first and second protrusions 34, 35 located at intervals. The depressed portions 38 and the protruded potions 39 are provided on the bridging portion 36 along the longitudinal direction of the loop 33 to form the longitudinal direction of the loop 33 into a corrugated shape. Accordingly, when the loop 33 is caught by the tongue papillae 40, the loop 33 is pulled to linearly extend the depressed and protruded potions 38, 39 and thus the loop slips through the tongue papillae 40 without being forced to be thrust into the tongue papillae 40, thus permitting the possibility of injuring the tongue papillae 40 to be reduced.

Further, the approximately central portion 36A in the longitudinal direction of the bridging portion 36 is formed into a circular-arc shape protruding upward. Hence, if the tongue papillae 40 are located at the end of the bridging portion, the tongue papillae 40 can move relatively toward the approximately central portion 36A of the bridging portion 36 during the movement of the loop 33 toward to the tongue, and therefore the length over which the loop 33 can slip through the tongue papillae 40 can be enlarged, so that the loop 33 can slip through the tongue papillae 40 without injuring the tongue papillae 40.

Furthermore, all of the first and second protrusions 34, 35 and the bridging portions 36 are provided with the depressed portions 38 and the protruded potions 39 along the longitudinal direction of the loops 33 to form the longitudinal portions of the loops 33 into a corrugated shape. Hence, the extendable rate at which the depressed portions 38 and the protruded potions 39 get linear to extend the loops 33 is increased as much as possible and as a result the length over which the loop 33 can slip through the tongue papillae 40 can be more enlarged, so that the loop 33 can slip through the tongue papillae 40 without injuring the same.

Besides, by using the loop pile as the loop 33, the loop 33 is allowed to be pressed against the surface of a tongue with an appropriate elastic force, so that the tongue cleaner can be used without injuring the surface of the tongue even more.

Moreover, according to the foregoing manufacturing method, as a method for forming the depressed portions 38 and the protruded potions 39, there is proposed the pressing of the rotating brush, for example, against the looped textile to apply a plastic processing so as to form depressed and protruded portions on the looped textile, so that a simplified manufacturing method can be realized.

### Fifth embodiment not forming part of the present invention

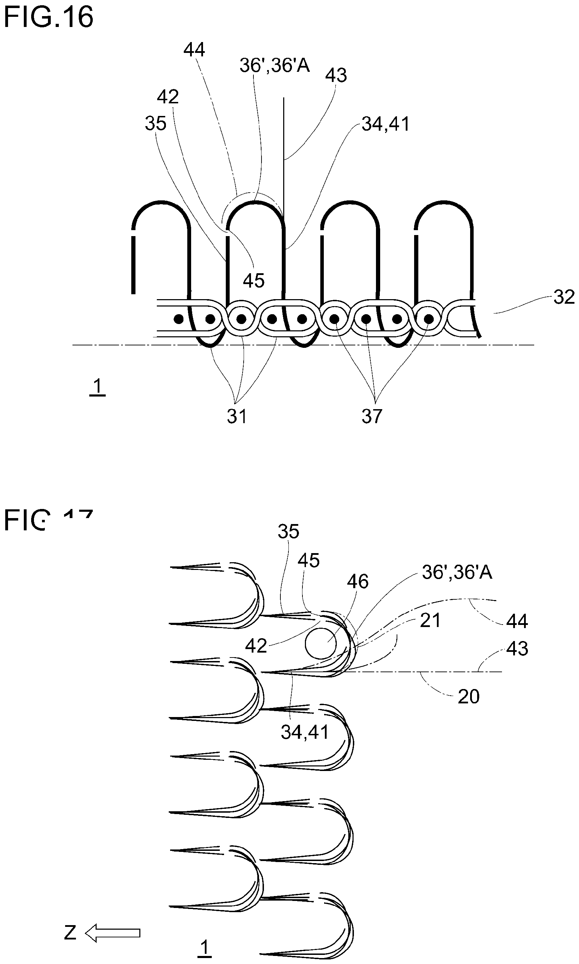

FIG. 16 and FIG. 17 show a fifth embodiment and specifically show a case where an improved pile-woven textile is used. A linear material 41 is provided so as to eliminate the continuity of any one of the first and second protrusions 34, 35 among these first and second protrusions 34, 35 and the bridging portion 36' forming the rising portion arranged between the distal ends thereof. According to the present embodiment, a disconnected portion 42 is formed on one side of the distal end of the second protrusion 35 and thus the first protrusion 34 and the bridging portion 36' are allowed to continuously extend in an inverted-J shape, whereas the second protrusion 35 is linearly formed. In addition, by forming the disconnected portion 42, the first and second protrusions 34, 35 are likely to get entangled with each other and therefore to prevent the occurrence of such tangle, a certain means (not shown) for preventing the linear material from coming away, such as bonding, welding or the like is desirably applied to the base textile 32.

As for an operation for forming the disconnected portion 42 in the loop 33, it is performed in the following manner. A cutter, e.g., is pressed against the looped textile 31 in a state of textile prior to being mounted on the head 1 as shown in FIG. 4 to thereby form the disconnected portion 42 in the textile 13. The one processed thus way is mounted on the head 1 as described above.

Accordingly, on a side of the distal end of the protrusion 34, a plurality of the protruding linear materials 41 provided on the main body of the cleaner are provided with the bridging portion 36', being the rising portion, formed so as to intersect with a first longitudinal direction 43 of the protrusion 34 to allow a second longitudinal direction 44 to face sideways, while a distal end 45 of the rising portion 36' is formed into a free end. An approximately central portion 36'A in the longitudinal direction 44 of the rising portion 36' is formed in a circular-arc shape protruding upward, while the distal end 45 of the rising portion 36' is folded toward the head 1 so that it is, as a whole, formed into the inverted-J shape. At this time, the width direction between the side of the distal end 45 and the protrusion 41 is defined so as to make a right angle with the moving direction Z.

As for a manufacturing method thereof, a cutting means such as a knife or the like (not shown) is applied to an upper end of the second protrusion 35 in the looped textile to form the disconnected portion 45.

Accordingly, when the head is allowed to enter a mouth with the stick-like handle held by hand to come in contact with a tongue and then the head 1 is moved along the moving direction Z, the linear material 41 is allowed to fall along the surface of a tongue, thus rubbing off the grime of tongue coating so as to scrape out the grime by the sideways rising portion 36' of the linear material 41.

When the grime of tongue coating is scraped out by moving the linear material 41 in the moving direction Z, and then when tongue papillae 46 thrust into the rising portion 36', e.g., and the rising portion 36' is caught by the tongue papillae 46, as shown in a dashed-dotted lines of FIG. 17, then, the angle between the first longitudinal direction 42 and the second longitudinal direction 43 increases so that the cantilevered rising portion is paralleled with the first longitudinal direction 42 by the tongue papillae 46 and as a result, the distal end 45 acting as a free end is allowed to slip through the tongue papillae 46, preventing the tongue papillae 46 from being injured.

As described above, according to the foregoing embodiment, on the side of the distal end of the protrusion 41, the linear material 41 is provided with the longitudinal part of the rising portion 36' in a direction intersecting with the longitudinal direction 42 of the protrusion 41 and the distal end 45 of the rising portion 36' is formed into a free end. Hence, when the rising portion 36' is caught by the tongue papillae 46, the rising portion 36' is pulled by the tongue papillae 46 to deform along the first longitudinal direction 42 of the protrusion 41 and thus the rising portion 36' can slip through the tongue papillae 46, so that the possibility of injuring the tongue papillae 46 can be reduced.

Further, when the longitudinal direction 43 of the rising portion 36' is provided sideways in relation to the longitudinal direction 42 of the protrusion 41 to clean tongue coating by moving the head 1, the rising portion 36' becomes sideways, so that the grime of the tongue coating can be surely rubbed out without failing to trap the grime of the tongue coating.

Furthermore, the side of the distal end 45 of the rising portion 36' is folded toward the head 1 and thus when the main body of the cleaner is moved in the direction Z to clean the tongue coating, the rising portion 36' becomes sideways and the distal end 45 is folded and therefore the tongue coating is enveloped with the protrusion 41, the rising portion 36' and the distal end 45 folded, so that the grime can be more surely rubbed off.

Besides, the approximately central portion 36'A in the longitudinal direction 43 of the rising portion 36' is formed into a circular-arc shape protruding upward and thus if the tongue papillae 46 is located at the end of the bridging portion 36', when the linear material 41 is moving relatively to a tongue, the tongue papillae 46 can move relatively to the approximately central portion 36'A of the rising portion 36'. Hence, the rising portion 36' is allowed to slip through the tongue papillae 46, so that the linear material 41 can slip through the tongue papillae 46 without injuring the tongue papillae 46.

### Sixth embodiment not forming part of the present invention

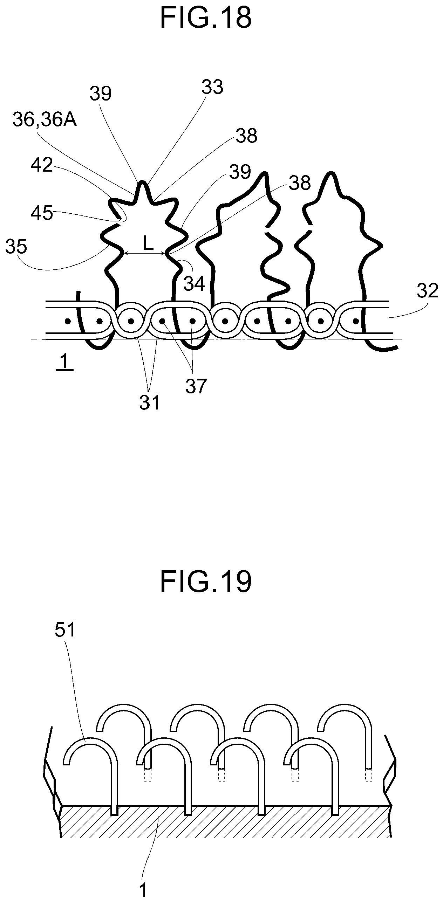

In a sixth embodiment shown in FIG. 18, the structures in the fifth and sixth embodiments are combined together. The disconnected portion 42 is formed in any one of the protrusions 34, 35 of the loop 33 provided continuously with depressed portions 38 and protruded portions 39 to make the distal end of a side of the bridging portion 36 forming the rising portion into a free end.

Accordingly, when the loop 33 is caught by the tongue papillae 46, the depressed portions 38 and the protruded portions 39 extend to act as a cushion and besides the distal ends 45 acting as a free end also deforms to the opposite side to the moving direction of the head 1, so that the tongue papillae 46 can be even more reliably prevented from being injured.

### Seventh embodiment not forming part of the present invention

In a seventh embodiment shown in FIG. 19, inverted-J-shaped linear materials 51 are provided on the head 1 in a protruding manner. Note that the linear materials 51 may be formed from animal hairs such as those of a horse, a pig or the like in addition to synthesized resin.

Accordingly, when the linear materials catch the tongue papillae, a side of the distal end in each of the linear materials is pulled by the tongue papillae 46 to deform and thus the linear materials can slip through the tongue papillae, causing an effect of being capable of reducing the possibility of injuring the tongue papillae.

As described above, the tongue cleaner may be modified in various ways, such as by varying its shapes. For example, the head may have a flat surface instead of having the raised and valley portions.

## Claims

1. A tongue cleaner provided with a plurality of looped linear materials (41) for scraping tongue coating in a protruding manner on a sheet (4) attached to a main body (1) of said tongue cleaner, wherein each of said looped linear materials includes first and second protrusions (34), (35) standing at an interval and rising from the sheet (4), and a bridging portion (36) provided between distal ends of said first and second protrusions (34), (35), and **characterized in that** depressed portions (38) and protruded portions (39) are provided along a longitudinal direction of each of said linear materials in at least part of said first and second protrusions (34), (35) and said bridging portion (36), such that each of said linear materials is formed into a corrugated shape.

2. The tongue cleaner according to claim 1, **characterized in that** each of said linear materials is a loop textile, and said depressed portions (38) and protruded portions (39) are formed by pressing of a rotating brush against said linear materials.

3. The tongue cleaner according to claim 1 or 2, **characterized in that** said depressed portions (38) and protruded portions (39) are maintained based on plastic deformation.

4. The tongue cleaner according to any one of claims 1 to 3, **characterized in that** an approximately central portion of said bridging portion (36) in a longitudinal direction is formed into a circular-arc shape protruding upward.

5. The tongue cleaner according to any one of claims 1 to 4, **characterized in that** said depressed portions (38) and protruded portions (39) are provided in said first and second protrusions (34) (35) and said bridging portion (36) along a longitudinal direction of each of said linear materials so as to form each of said linear materials into a corrugated shape.

6. The tongue cleaner according to any one of claims 1 to 5, **characterized in that** each of said linear materials is a loop pile.

## Patentansprüche

1. Ein Zungenreiniger, der mit einer Vielzahl von schlaufenförmigen linearen Materialien (41) zum Abstreifen einer Zungenbeschichtung in vorstehender Weise auf einen an einem Grundkörper (1) dieses Zungenreinigers angebrachten Bogen (4) versehen ist,
wobei jedes dieser schlaufenförmigen linearen Materialien erste und zweite Vorsprünge (34), (35), die in einem Abstand angeordnet sind und von dem Bogen (4) hochstehen, und einen Brückenabschnitt (36) aufweist, der zwischen distalen Enden der ersten und der zweiten Vorsprünge (34), (35) vorgesehen ist, und
**dadurch gekennzeichnet, dass** vertiefte Abschnitte (38) und vorstehende Abschnitte (39) entlang einer Längsrichtung von jedem der linearen Materialien in zumindest einem Teil der ersten und der zweiten Vorsprünge (34), (35) und dem Überbrückungsabschnitt (36) vorgesehen sind, so dass jedes der linearen Materialien in eine gewellte Form gebracht ist.

2. Der Zungenreiniger nach Anspruch 1, **dadurch gekennzeichnet, dass** jedes der linearen Materialien ein Schlingentextil ist, und dass die vertieften Abschnitte (38) und die vorstehenden Abschnitte (39) durch Drücken einer rotierenden Bürste gegen die linearen Materialien gebildet sind.

3. Der Zungenreiniger nach Anspruch 1 oder 2, **dadurch gekennzeichnet, dass** die vertieften Abschnitte (38) und die vorstehenden Abschnitte (39) basierend auf einer plastischen Verformung aufrechterhalten werden.

4. Der Zungenreiniger nach einem der Ansprüche 1 bis 3, **dadurch gekennzeichnet, dass** ein etwa mittlerer Abschnitt des Brückenabschnitts (36) in einer Längsrichtung zu einer nach oben hervorstehenden Kreisbogenform ausgebildet ist.

5. Der Zungenreiniger nach einem der Ansprüche 1 bis 4, **dadurch gekennzeichnet, dass** die vertieften Abschnitte (38) und die vorstehenden Abschnitte (39) in den genannten ersten und zweiten Vorsprüngen (34) (35) und dem genannten Brückenabschnitt (36) entlang einer Längsrichtung jedes der linearen Materialien vorgesehen sind, um jedes der linearen Materialien in eine gewellte Form zu formen.

6. Der Zungenreiniger nach einem der Ansprüche 1 bis 5, **dadurch gekennzeichnet, dass** jedes der linearen Materialien ein Schlingenflor ist.

## Revendications

1. Dispositif de nettoyage de la langue doté d'une pluralité de matériaux linéaires en boucle (41) pour racler le revêtement de la langue en saillie sur une feuille (4) fixée à un corps principal (1) dudit dispositif de nettoyage de la langue,
dans lequel
chacun desdits matériaux linéaires en boucle comprend des première et seconde saillies (34), (35) dressées à un intervalle et s'élevant à partir de la feuille (4), et une partie de pontage (36) fournie entre les extrémités distales desdites première et seconde saillies (34), (35), et
**caractérisé en ce que**
des parties renfoncées (38) et des parties en saillie (39) sont fournies dans une direction longitudinale de chacun desdits matériaux linéaires dans au moins une partie desdites première et seconde saillies (34), (35) et ladite partie de pontage (36), de telle sorte que chacun desdits matériaux linéaires ait une forme ondulée.

2. Dispositif de nettoyage de la langue selon la revendication 1, **caractérisé en ce que** chacun desdits matériaux linéaires est un textile à boucles, et lesdites parties renfoncées (38) et parties en saillie (39) sont formées par pression d'une brosse rotative contre lesdits matériaux linéaires.

3. Dispositif de nettoyage de la langue selon la revendication 1 ou 2, **caractérisé en ce que** lesdites parties renfoncées (38) et parties en saillie (39) sont maintenues sur la base d'une déformation plastique.

4. Dispositif de nettoyage de la langue selon l'une quelconque des revendications 1 à 3, **caractérisé en ce qu'**une partie approximativement centrale de ladite partie de pontage (36) dans une direction longitudinale a une forme d'arc circulaire en saillie vers le haut.

5. Dispositif de nettoyage de la langue selon l'une quelconque des revendications 1 à 4, **caractérisé en ce que** lesdites parties renfoncées (38) et parties en saillies (39) sont fournies dans lesdites première et seconde saillies (34) (35) et ladite partie de pontage (36) dans une direction longitudinale de chacun desdits matériaux linéaires pour mettre chacun desdits matériaux linéaires sous une forme ondulée.

6. Dispositif de nettoyage de la langue selon l'une quelconque des revendications 1 à 5, **caractérisé en ce que** chacun desdits matériaux linéaires est un velours bouclé.
